# EUROPÄISCHE PATENTSCHRIFT

(11) **EP 3 011 978 B1**
(45) Veröffentlichungstag und Bekanntmachung des Hinweises auf die Patenterteilung: **10.05.2017**
(21) Anmeldenummer: 14190214.8
(22) Anmeldetag: 24.10.2014
(51) Int. Cl.: A61L 15/22, A61L 15/42, A61F 13/02

(54) **Wundauflagematerial und Verfahren zu seiner Herstellung**
Wound dressing material and method for producing the same
Matériau pour pansement et son procédé de fabrication

(43) Veröffentlichungstag der Anmeldung: 27.04.2016
(73) Patentinhaber: Sefar AG, 9410 Heiden (CH)
(72) Erfinder: Gerdes, Gerd, 9030 Abtwil (CH); Weber, Jétémie, 6833 Klaus (AT); Häusser, Alexander, 8280 Kreuzlingen (CH)
(74) Vertreter: Wunderlich, Rainer

(56) Entgegenhaltungen:
- US-A- 4 984 570
- US-A1- 2002 052 570
- US-A1- 2013 150 764

## Beschreibung

Die Erfindung betrifft ein Wundauflagematerial zum Auflegen auf eine Wunde, insbesondere eines Menschen, gemäß dem Oberbegriff des Anspruchs 1. Die Erfindung betrifft weiterhin ein Verfahren zum Herstellen eines solchen Wundauflagemateriales gemäß Anspruch 13.

Derartige Wundmaterialien sind als streifenförmige Verbandmaterialien oder als Pflaster seit langem bekannt. Üblicherweise bestehen Verbandmaterialien aus Baumwollstoff oder einem anderen Fasermaterial, welches ein gewisses Absorptionsvermögen besitzt. Dieses ist in vielen Fällen gewünscht, da so das Wundauflagematerial eine die Heilung der Wunde unterstützende Salbe oder Flüssigkeit aufnehmen und speichern oder zu einem Trockenhalten der Wunde Wundsekret aufnehmen kann.

Allerdings besteht die Problematik bei saugfähigen Fasermaterialien, dass diese anfällig zur Aufnahme von Keimen sind. Derartige Verbandmaterialien sind daher für eine gute Wundheilung relativ häufig zu wechseln.

Ein häufiges Wechseln des Verbandmateriales kann aber selbst die Wundheilung negativ beeinflussen, insbesondere dann, wenn Gewebematerial an der Wundauflage anhaftet und beim Wechseln die Wunde aufgerissen wird.

Es ist weiter bekannt, als Wundauflagematerial auch ein Monofilamentgewebe vorzusehen. Allerdings weist ein derartiges Gewebe kaum ein Absorptionsvermögen für Flüssigkeiten auf, wie es für einen Wundverband gewünscht ist.

Aus der US 4,984,570 A geht ein Wundauflagematerial hervor, bei dem eine Auflageseite aus synthetischen Fäden zusammen mit natürlichen Fäden zum Bilden eines Aufnahmebereiches für Wundsekret durch Wirken einer Maschenstruktur hergestellt ist. Durch Falten des einstückig gewirkten Textilmateriales wird ein Schichtaufbau erzeugt.

Die gattungsbildende US 2002/0052570 A1 lehrt einen elastischen, selbsthaftenden Verbandstreifen mit einem Pad zum Absorbieren von Wundsekret. An einer die Wunde kontaktierenden Auflageseite des Pads ist eine nicht-haftende Auflageschicht vorgesehen.

Der Erfindung liegt die **Aufgabe** zugrunde, ein Wundauflagematerial anzugeben, welches für eine gute Wundheilung besonders förderlich ist. Weiterhin ist es Aufgabe der Erfindung, ein Verfahren zur Herstellung eines derartigen Wundauflagemateriales anzugeben.

Die Aufgabe wird zum einen durch ein Wundauflagematerial mit den Merkmalen des Anspruchs 1 und zum anderen durch ein Verfahren mit den Merkmalen des Anspruchs 13 gelöst. Bevorzugte Ausführungsformen sind in den abhängigen Ansprüchen angegeben.

Gemäß der Erfindung ist ein Wundauflagematerial mit einem biokompatiblen Monofilamentgewebe vorgesehen, welches eine Auflageseite auf eine Wunde bildet und als eine Trägerschicht ausgebildet ist, auf welcher ein Deckelement aufgebracht und dieses zumindest bereichsweise fest mit der Trägerschicht verbunden ist.

Ein Aspekt der Erfindung besteht in einem mehrschichtigen Aufbau des Wundauflagemateriales, wobei eine Auflageseite auf die Wunde durch ein biokompatibles Monofilamentgewebe gebildet ist. Das Monofilamentgewebe ist aus Monofilamentfäden, also aus Kunststoffdrähten, gebildet. Diese Monofilamentfäden werden mit einer glatten, stabilen Oberfläche gebildet, so dass im Gegensatz zu Multifilamentfäden, kein Fasermaterial von der Oberseite der Fäden in die Wunde eindringen kann. Zudem wird durch die glatte Oberfläche eine geringere Anhaftung von Gewebematerial der Wunde an der Auflageseite des Wundauflagemateriales bewirkt. Somit wird der Gefahr eines Aufreißens der Wunde bei einem Lösen des Wundauflagemateriales von der Wunde entgegengewirkt. Dies ist schonend für einen Patienten und fördert die Wundheilung.

Gemäß einem weiteren Aspekt der Erfindung ist das Monofilamentgewebe biokompatibel ausgebildet. Die Auswahl der Rohstoffe und die Verarbeitung zu dem Monofilamentgewebe ist dabei so gestaltet, dass das Monofilamentgewebe nicht irritierend ist, insbesondere nicht zytotoxisch, pyrogenfrei und hypoallergen ist. Gewebe, wie sie üblicherweise in der Industrie eingesetzt sind, sind grundsätzlich nicht biokompatibel. Biokompatible Gewebe sind aber aus dem Bereich der Blutfiltration bekannt. Von einer Biokompatibilität des Gewebes kann insbesondere dann ausgegangen werden, wenn die Anforderungen nach ISO 10993 oder des "Class VI-Test" der USP (United States Pharmacopeia) erfüllt sind.

Durch die Biokompatibilität wird erreicht, dass einerseits eine Irritation des verletzten Gewebemateriales durch das Wundauflagematerial weitgehend vermieden wird und andererseits beim Ablösen des Wundauflagemateriales die Gefahr einer Beeinträchtigung, insbesondere eines Aufreißens, der Wunde entgegengewirkt ist.

Darüber hinaus ist gemäß einem weiteren Aspekt der Erfindung das biokompatible Monofilamentgewebe so stabil ausgebildet, dass es als eine Trägerschicht dient, auf welcher mindestens ein Deckelement fest angebracht ist. Dieses Deckelement kann dabei selbst eine einzelne Schicht oder mehrere Schichten aufweisen. Entsprechend dem Einsatzzweck des Wundauflagemateriales kann das Deckelement in einer gewünschten Weise, etwa zum Aufnehmen von Wundsekret, oder zum Aufbringen von wundheilungsfördernden Mitteln, etwa einer Salbe oder einer Flüssigkeit, ausgebildet sein.

Gemäß der Erfindung können verschiedene Elemente und Materialien zum Bilden des Deckelementes vorgesehen sein, da diese auf der von der Auflageseite des Monofilamentgewebes abgewandten Oberseite angeordnet sind und somit nicht unmittelbar mit dem Gewebematerial im Wundbereich in Kontakt gelangen. Das Deckelement kann selbst einen mehrschichtigen Aufbau aufweisen.

Eine bevorzugte Ausführungsform der Erfindung besteht darin, dass das Monofilamentgewebe als ein Einfachgewebe mit offenmaschiger Struktur ausgebildet ist, welches eine Porengröße von 5 µm bis 500 µm, vorzugsweise 20 µm bis 300 µm, aufweist, und dass die Poren einen Anteil von 15% bis 70% der Fläche des Monofilamentgewebes bilden. Das erfindungsgemäße Monofilamentgewebe ist gemäß dieser Ausführungsform also besonders feinporig, so dass zwar einerseits ein Luft- und Flüssigkeitsdurchtritt durch die Poren möglich ist, jedoch andererseits ein Verwachsen oder Umgreifen des Monofilamentgewebes durch Gewebematerial kaum möglich ist. Es wird so eine schützende, aber durchlässige Wundauflage geschaffen, welche jedoch weiterhin eine klare Abgrenzung und damit eine leichte Lösbarkeit von der Wunde sicherstellt. Die gute Durchlässigkeit für Luft und Flüssigkeit ist insbesondere dadurch gegeben, dass die Poren einen Anteil von 15% bis 70% der Fläche des Monofilamentgewebes, vorzugsweise 40% bis 70%, bilden. Auf diese Weise wird eine hohe Durchlässigkeit erreicht.

Dabei ist das Monofilamentgewebe als ein Einfachgewebe ausgebildet, welches eine im Wesentlichen zweidimensionale Gewebekonstruktion mit einem einzelnen Kettfadensystem und einem einzelnen Schussfadensystem aufweist. Durch die Verwendung eines transparenten Fadenmateriales für die Monofilamentfäden wird zudem in Kombination mit der großen Porenfläche eine sehr hohe Transparenz erreicht. Trotz einer relativ dünnen Gewebedicke kann eine ausreichende Stabilität der durch das Monofilamentgewebe gebildeten Trägerschicht bei einer guten Gewebeoffenheit durch eine Leinwandbindung ein Taffet 1:1 erreicht werden. Eine weitere stabile Konstruktion kann durch eine Köperbindung in beliebigen Rapporten, bevorzugt in einer Bindung 1:2, 1:3, 2:2 und 3:3 betreffend Kettfaden-/Schussfaden-Anordnung, erreicht werden. Eine weitere stabile Bindung ist durch eine Atlasbindung in beliebigen Rapporten möglich, besonders bevorzugt in einer Atlasbindung 1:7 betreffend Kettfaden-/Schussfaden-Anordnung. Eine weitere mögliche stabile Bindung für das Monofilamentgewebe ist eine Tressenbindung.

Gemäß der Erfindung ist es vorgesehen, dass die Monofilamentfäden des Monofilamentgewebes aus PET gebildet sind und eine Durchmessergröße zwischen 20 µm bis 500 µm, vorzugsweise zwischen 30 µm bis 150 µm, aufweisen. Die Monofilamentfäden sind dabei aus besonders reinem Ausgangsmaterial hergestellt, so dass diese lebensmittelkonform sind. Es können pyrogenfreie und nicht zytotoxische Gewebe hergestellt werden. Die Fadendurchmesser können je nach Anwendungsfall variieren, sind jedoch vorzugsweise möglichst klein gehalten. Insbesondere sind die Monofilamentfäden mit einer Längsrippenstruktur ausgebildet, bei welchen die Längsrippen von einem Kerndurchmesser des Monofilamentfadens höchstens 0,5 µm bis 5 µm radial vorstehen. Durch diese sehr feine Rippenausbildung wird weiterhin eine relativ glatte Oberfläche der Monofilamentfäden gewährleistet, während gleichzeitig eine hinreichende Stabilität an den Kreuzungspunkten der Monofilamentfäden im Gewebe und damit eine stabile Formgebung der Poren gegeben ist. Zusätzlich kann das Monofilamentgewebe kalandriert sein.

Eine weitere bevorzugte Ausführungsform der Erfindung besteht darin, dass die Auflageseite zumindest bereichsweise mit einer Haftschicht versehen ist. Die Haftschicht ist dabei üblicherweise ein Klebstoffauftrag, welcher zum Aufbringen auf Haut geeignet ist. Die Haftschicht kann dabei unmittelbar auf der Auflageseite des Monofilamentgewebes aufgebracht werden.

Diese Ausführung der Erfindung ist in bevorzugter Weise dadurch weitergebildet, dass die Haftschicht streifenförmig entlang eines Randbereiches der Auflageseite des Monofilamentgewebes aufgebracht ist und dass die von der Auflageseite abgewandte Oberseite des Randbereiches des Monofilamentgewebes von dem Deckelement freigehalten ist.

Bei dieser Ausführungsform erstreckt sich also das Deckelement nicht über die gesamte Oberfläche des Monofilamentgewebes, sondern lediglich in einem Mittenbereich, während ein streifenförmiger Randbereich freigehalten ist. Der Randbereich kann sich nur entlang eines Teilbereiches des Außenumfanges erstrecken oder entlang des gesamten Außenumfanges ausgebildet und damit ringförmig geschlossen sein. Durch diese streifenförmige Haftschicht wird eine Fixierung des Wundauflagemateriales beabstandet zur Wunde ermöglicht. Durch ein Freihalten der Oberseite des Randbereiches wird ein hinreichender Luftzutritt ermöglicht und einer Irritation der Haut durch die Haftschicht entgegengewirkt.

Der Randbereich des Wundauflagemateriales ist besäumt, insbesondere durch eine thermische Besäumung (Heißschnitt), bei welcher die Schnittkanten verschweißt und Schneidkanten abgerundet werden. Dabei kann die Haftschicht auch auf einem Auflageelement aufgebracht sein, welches wiederum entlang des Randbereiches des Monofilamentgewebes aufgebracht ist. Dieses Auflageelement kann dabei um die Randkante des Monofilamentgewebes umgeschlagen sein und dieses zusätzlich umsäumen, um so einen Tragekomfort weiter zu erhöhen.

Gemäß einer Weiterbildung der Erfindung ist es vorteilhaft, dass das Deckelement ein Gewebe, ein non-woven Textil, eine Folie, eine Membran und/oder einen Schaum aufweist. Das Deckelement kann insbesondere selbst einen schichtförmigen oder mehrlagigen Aufbau aufweisen, wobei die einzelnen Schichten oder Lagen selbst aus gleichen oder unterschiedlichen Materialien gebildet sind. Bei der Verwendung eines Gewebes kann insbesondere neben einem weiteren Monofilamentgewebe auch ein Multifilamentgewebe mit einer erhöhten Saugfähigkeit Anwendung finden. Zu diesem Zweck können auch non-woven Textilien, also etwa ein Gewirk, ein Vlies, ein Filz oder ähnliches oder auch Watte zum Einsatz kommen. Diese Materialien sind auch gut geeignet, pflegende oder medizinisch wirksame Substanzen aufzunehmen. Für einen Schutz gegen den Zutritt von Flüssigkeiten, Gasen oder weiteren physikalischen Einwirkungen von außen kann eine Folie, eine Membran und/oder ein Schaum vorgesehen sein. Die Membran kann dabei vorzugsweise semipermeabel sein, so dass diese zwar einen Gasaustausch zulässt, jedoch keinen Eintritt von Flüssigkeit von außen in die Wunde oder umgekehrt von Flüssigkeit aus der Wunde in einen Außenbereich.

Nach der Erfindung ist es vorgesehen, dass das Deckelement mit dem Monofilamentgewebe punktverschweißt ist. Dies kann über ein thermisches Verschweißen oder vorzugsweise über ein Ultraschallverschweißen erfolgen. Das Verschweißen kann auch bei einem Heißzuschnitt oder Laserschnitt im Randbereich erfolgen. Das Deckelement kann entlang des Randbereiches linienförmig mit dem Monofilamentgewebe verschweißt sein, so dass das Deckelement an seiner Außenseite insbesondere umfassend oder ringförmig geschlossen ist.

Eine weitere vorteilhafte Ausführungsform der Erfindung besteht darin, dass das Deckelement eine äußere Deckschicht aufweist, welche mit dem Monofilamentgewebe unter Ausbildung mindestens eines Aufnahmebereiches verbunden ist. Der mindestens eine Aufnahmebereich kann dabei ein Hohlraum sein, in welchem ein weiteres Element zur Aufnahme von Flüssigkeit oder einer Substanz vorgesehen ist. Die äußere Deckschicht kann dabei insbesondere eine Folie oder eine Membran sein, welche einen flüssigkeitsdichten Abschluss nach außen bildet. Hierdurch kann insbesondere erreicht werden, dass auf dem Wundauflagematerial getragene Bekleidungsstücke nicht durch Substanzen aus dem Wundauflagematerial oder durch Wundsekrete verschmutzt werden.

Zur Reduzierung der Gewebeirritation ist es nach einer weiteren Ausführungsvariante der Erfindung vorgesehen, dass die Dicke der durch das Deckelement gebildeten Oberseite 60% bis 98% der Gesamtdicke des Wundauflagemateriales beträgt. Das biokompatible Monofilamentgewebe ist dabei sehr fein und weist eine Dicke von 40 µm bis 1 mm, vorzugsweise zwischen 70 µm bis 300 µm auf. Das Deckelement, welches die Oberseite des Wundauflagemateriales bildet, kann abhängig vom Anwendungsfall zwischen 60 µm bis zu einigen Millimetern betragen.

Zur Förderung der Wundheilung ist es gemäß einer weiteren erfindungsgemäßen Ausführungsform vorgesehen, dass das Deckelement eine pflegende oder medizinisch wirksame Substanz aufweist. Das Deckelement kann dabei ein Reservoir mit einer Flüssigkeits- oder Gelfüllung oder zur Aufnahme von Pulvern oder Feststoffen ausgebildet sein.

Grundsätzlich ist es vorgesehen, dass die Monofilamentfäden aus einem Kunststoffmaterial mit einer hohen Reinheit und so gefertigt werden, dass diese von alleine eine möglichst glatte Oberfläche aufweisen. Eine vorteilhafte Ausführungsform der Erfindung besteht darin, dass das Monofilamentgewebe oder die Monofilamentfäden, welche das Monofilamentgewebe bilden, mit einer Beschichtung versehen sind. Mit dieser Beschichtung kann die Oberfläche in einer gewünschten Weise behandelt und beeinflusst werden. Insbesondere sind eine hydrophile oder eine hydrophobe Beschichtung möglich. Zudem kann die Rauigkeit und die Anhafteigenschaft der Oberfläche weiter reduziert werden. Neben einer derartigen Oberflächenbeschichtung mit geeigneten Materialien kann zudem auch eine andere Oberflächenmodifikation, insbesondere eine Glättung der Fadenoberflächen oder der Oberfläche des fertigen Monofilamentgewebes eingestellt werden.

Eine weitere vorteilhafte Ausführung der Erfindung kann darin gesehen werden, dass das Monofilamentgewebe mit einer gleichmäßigen definierten Porengröße gewebt ist, wobei die Porengrößen nicht mehr als 10%, vorzugsweise weniger als 5% voneinander abweichen. Es wird also ein Monofilamentgewebe geschaffen, welches über die gesamte Fläche eine sehr gleichmäßige Porengröße aufweist. Abhängig vom Einsatzzweck kann so eine möglichst zweckmäßige Porengröße vorgesehen werden, welche einerseits etwa einen Luft- oder Flüssigkeitsdurchtritt in gewünschter Weise ermöglicht, während andererseits die Gefahr eines Einwachsens oder Umgreifens von Gewebematerial weitgehend entgegengewirkt ist.

Nach der Erfindung ist ein Verfahren zum Herstellen eines Wundauflagemateriales, insbesondere wie es zuvor beschrieben worden ist, vorgesehen, bei dem ein biokompatibles Monofilamentgewebe als ein erstes Bahnmaterial gebildet wird, ein Deckelement als ein zweites Bahnmaterial gebildet wird, das erste Bahnmaterial und das zweite Bahnmaterial zusammengeführt werden, wobei das biokompatible Monofilamentgewebe und das Deckelement fest miteinander verbunden, insbesondere verschweißt werden, und das Wundauflagematerial als ein Band hergestellt wird. Das so hergestellte Band kann zu einer Rolle aufgespult oder in gewünschter Weise beschnitten und konfektioniert werden.

Das erfindungsgemäße Verfahren ermöglicht eine effiziente Herstellung des Wundauflagemateriales in einem kontinuierlichen Verfahren. Die Ausgangsmaterialien werden dabei vorzugsweise von Rollen als bandförmige Bahnen zugeführt. Das Deckelement kann dabei ein vorgefertigtes Bahnmaterial sein, welches aus mehreren Lagen besteht. Es ist aber ebenso möglich, dass das Deckelement aus mehreren Bahnmaterialien gebildet wird, welche gleichzeitig zugeführt und mit dem Monofilamentgewebe verbunden werden.

## Patentansprüche

1. Wundauflagematerial mit einem Deckelement zum Aufnehmen von Wundsekret oder zum Aufbringen von wundheilungsfördernden Mitteln und einer Auflageseite zum Auflegen auf die Wunde,
**dadurch gekennzeichnet,**
- **dass** die Auflageseite durch ein biokompatibles Monofilamentgewebe aus Monofilamentfäden aus PET mit einer glatten stabilen Oberfläche gebildet ist,
- **dass** die PET-Monofilamentfäden eine Durchmessergröße zwischen 20 µm bis 500 µm aufweisen, und
- **dass** das Monofilamentgewebe als eine Trägerschicht mit einer von der Wunde abgewandten Oberseite ausgebildet ist, auf welcher das Deckelement aufgebracht und durch Punktverschweißen bereichsweise fest mit der Trägerschicht verbunden ist.

2. Wundauflagematerial nach Anspruch 1,
**dadurch gekennzeichnet,**
**dass** das Monofilamentgewebe als ein Einfachgewebe mit offenmaschiger Struktur gebildet ist, welches eine Porengröße von 5 µm bis 500 µm, vorzugsweise 20 µm bis 300 µm, aufweist, und
**dass** die Poren einen Anteil von 15% bis 60% der Fläche des Monofilamentgewebes bilden.

3. Wundauflagematerial nach Anspruch 1 oder 2,
**dadurch gekennzeichnet,**
**dass** die Monofilamentfäden des Monofilamentgewebes eine Durchmessergröße zwischen 50 µm bis 150 µm aufweisen.

4. Wundauflagematerial nach einem der Ansprüche 1 bis 3,
**dadurch gekennzeichnet,**
**dass** die Auflageseite zumindest bereichsweise mit einer Haftschicht versehen ist.

5. Wundauflagematerial nach Anspruch 4,
**dadurch gekennzeichnet,**
**dass** die Haftschicht streifenförmig entlang eines Randbereiches der Auflageseite des Monofilamentgewebes aufgebracht ist und
**dass** die von der Auflageseite abgewandte Oberseite des Randbereiches des Monofilamentgewebes von dem Deckelement freigehalten ist.

6. Wundauflagematerial nach einem der Ansprüche 1 bis 5,
**dadurch gekennzeichnet,**
**dass** das Deckelement ein Gewebe, ein non-woven Textil, eine Folie, eine Membran und/oder einen Schaum aufweist.

7. Wundauflagematerial nach einem der Ansprüche 1 bis 6,
**dadurch gekennzeichnet,**
**dass** das Deckelement mit dem Monofilamentgewebe durch Ultraschallverschweißen punktverschweißt ist.

8. Wundauflagematerial nach einem der Ansprüche 1 bis 7,
**dadurch gekennzeichnet,**
**dass** das Deckelement eine äußere Deckschicht aufweist, welche mit dem Monofilamentgewebe unter Ausbildung mindestens eines Aufnahmebereiches verbunden ist.

9. Wundauflagematerial nach einem der Ansprüche 1 bis 8,
**dadurch gekennzeichnet,**
**dass** die Dicke der durch das Deckelement gebildeten Oberseite 60% bis 98% der Gesamtdicke des Wundauflagemateriales beträgt.

10. Wundauflagematerial nach einem der Ansprüche 1 bis 9,
**dadurch gekennzeichnet,**
**dass** das Deckelement eine pflegende oder medizinisch wirksame Substanz aufweist.

11. Wundauflagematerial nach einem der Ansprüche 1 bis 10,
**dadurch gekennzeichnet,**
**dass** das Monofilamentgewebe oder die Monofilamentfäden, welche das Monofilamentgewebe bilden, mit einer Beschichtung versehen sind.

12. Wundauflagematerial nach einem der Ansprüche 1 bis 11,
**dadurch gekennzeichnet,**
**dass** das Monofilamentgewebe mit einer gleichmäßigen definierten Porengröße gewebt ist, wobei die Porengrößen nicht mehr als 10% voneinander abweichen.

13. Verfahren zum Herstellen eines Wundauflagemateriales nach einem der Ansprüche 1 bis 12, bei dem
- ein biokompatibles Monofilamentgewebe aus Monofilamentfäden aus PET mit einer glatten stabilen Oberfläche als ein erstes Bahnmaterial gebildet wird, wobei die PET-Monofilamentfäden eine Durchmessergröße zwischen 20 µm bis 500 µm aufweisen,
- ein Deckelement als ein zweites Bahnmaterial gebildet wird,
- das erste Bahnmaterial und das zweite Bahnmaterial zusammengeführt werden, wobei das biokompatible Monofilamentgewebe als eine Trägerschicht mit einer von der Wunde abgewandten Oberseite vorgesehen wird, auf welcher das Deckelement aufgebracht und durch Punktverschweißen fest mit der Trägerschicht verbunden wird, und
- das Wundauflagematerial als ein Band hergestellt wird.

## Claims

1. Wound dressing material having a cover element for receiving wound secretions or for applying means promoting wound healing and a contact side for placing onto the wound,
**characterized in that**
- the contact side is formed by a biocompatible monofilament fabric consisting of monofilament threads of PET with a smooth stable surface,
- the PET-monofilament threads have a diameter size ranging between 20 µm and 500 µm, and
- **in that** the monofilament fabric is designed as a carrier layer with an upper side which faces away from the wound and onto which the cover element is applied and firmly connected in some areas to the carrier layer through spot welding.

2. Wound dressing material according to claim 1,
**characterized in that**
the monofilament fabric is formed as a single-weave fabric with an open-mesh structure having a pore size ranging from 5 µm to 500 µm, preferably from 20 µm to 300 µm, and
**in that** the pores constitute a proportion of 15% to 60% of the surface of the monofilament fabric.

3. Wound dressing material according to claim 1 or 2,
**characterized in that**
the monofilament threads of the monofilament fabric have a diameter size ranging between 50 µm and 150 µm.

4. Wound dressing material according to any one of claims 1 to 3,
**characterized in that**
the contact side is provided at least in some areas with an adhesive layer.

5. Wound dressing material according to claim 4,
**characterized in that**
the adhesive layer is applied in a strip-shaped manner along a marginal area of the contact side of the monofilament fabric and
**in that** the upper side of the marginal area of the monofilament fabric which faces away from the contact side is kept free from the cover element.

6. Wound dressing material according to any one of claims 1 to 5,
**characterized in that**
the cover element has a fabric, a non-woven textile, a film, a membrane and/or a foam.

7. Wound dressing material according to any one of claims 1 to 6,
**characterized in that**
the cover element is spot-welded to the monofilament fabric through ultrasonic welding.

8. Wound dressing material according to any one of claims 1 to 7,
**characterized in that**
the cover element has an external cover layer which is connected to the monofilament fabric by forming at least one receiving area.

9. Wound dressing material according to any one of claims 1 to 8,
**characterized in that**
the thickness of the upper side formed by the cover element amounts to 60% to 98% of the overall thickness of the wound dressing material.

10. Wound dressing material according to any one of claims 1 to 9,
**characterized in that**
the cover element has a care substance or medically effective substance.

11. Wound dressing material according to any one of claims 1 to 10,
**characterized in that**
the monofilament fabric or the monofilament threads that form the monofilament fabric are provided with a coating.

12. Wound dressing material according to any one of claims 1 to 11,
**characterized in that**
the monofilament fabric is woven with a uniform defined pore size, wherein the pore sizes do not differ from each other by more than 10%.

13. Method for producing a wound dressing material according to any one of claims 1 to 12, in which
- a biocompatible monofilament fabric consisting of monofilament threads of PET with a smooth stable surface is formed as a first web material, wherein the PET-monofilament threads have a diameter size ranging between 20 µm and 500 µm,
- a cover element is formed as a second web material,
- the first web material and the second web material are joined, wherein the biocompatible monofilament fabric is provided as a carrier layer with an upper side which faces away from the wound and onto which the cover element is applied and firmly connected to the carrier layer through spot welding, and
- the wound dressing material is produced as a tape.

## Revendications

1. Matériau pour pansement avec un élément de recouvrement servant à absorber des sécrétions de plaies ou servant à appliquer des produits favorisant la cicatrisation des plaies et une face de pose destinée à être posée sur la plaie,
**caractérisé en ce**
- **que** la face de pose est formée par un tissu de monofilaments biocompatible avec une surface stable lisse composé de fils de monofilament en PET,
- **que** les fils de monofilament en PET présentent un diamètre de dimensions comprises entre 20 µm et 500 µm, et
- **que** le tissu de monofilaments est réalisé en tant que couche de support avec une face supérieure opposée à la plaie, sur laquelle l'élément de recouvrement est appliqué et est relié de manière solidaire à la couche de support par endroits par un soudage par points.

2. Matériau pour pansement selon la revendication 1,
**caractérisé en ce**
**que** le tissu de monofilaments est formé sous la forme d'un tissu simple avec une structure à mailles ouvertes, lequel présente des pores de dimensions allant de 5 µm à 500 µm, de préférence de 20 µm à 300 µm, et
**que** les pores forment une proportion allant de 15 % à 60 % de la surface du tissu de monofilaments.

3. Matériau pour pansement selon la revendication 1 ou 2,
**caractérisé en ce**
**que** les fils de monofilaments du tissu de monofilaments présentent un diamètre de dimensions comprises entre 50 µm et 150 µm.

4. Matériau pour pansement selon l'une quelconque des revendications 1 à 3,
**caractérisé en ce**
**que** la face de pose est pourvue au moins par endroits d'une couche adhésive.

5. Matériau pour pansement selon la revendication 4,
**caractérisé en ce**
**que** la couche adhésive est appliquée sous la forme d'un ruban le long d'une zone de bord de la face de pose du tissu de monofilaments, et
**que** la face supérieure, opposée à la face de pose, de la zone de bord du tissu de monofilaments reste exempte de l'élément de recouvrement.

6. Matériau pour pansement selon l'une quelconque des revendications 1 à 5,
**caractérisé en ce**
**que** l'élément de recouvrement présente un tissu, un textile non-tissé, un film, une membrane et/ou une mousse.

7. Matériau pour pansement selon l'une quelconque des revendications 1 à 6,
**caractérisé en ce**
**que** l'élément de recouvrement est soudé par points au tissu de monofilaments par un soudage par ultrasons.

8. Matériau pour pansement selon l'une quelconque des revendications 1 à 7,
**caractérisé en ce**
**que** l'élément de recouvrement présente une couche de recouvrement extérieure, qui est reliée au tissu de monofilaments en réalisant au moins une zone d'absorption.

9. Matériau pour pansement selon l'une quelconque des revendications 1 à 8,
**caractérisé en ce**
**que** l'épaisseur de la face supérieure formée par l'élément de recouvrement représente 60 % à 98 % de l'épaisseur totale du matériau pour pansement.

10. Matériau pour pansement selon l'une quelconque des revendications 1 à 9,
**caractérisé en ce**
**que** l'élément de recouvrement présente une substance active de soin ou à action médicale.

11. Matériau pour pansement selon l'une quelconque des revendications 1 à 10,
**caractérisé en ce**
**que** le tissu de monofilaments ou les fils de monofilaments, qui forment le tissu de monofilaments, sont pourvus d'un revêtement.

12. Matériau pour pansement selon l'une quelconque des revendications 1 à 11,
**caractérisé en ce**
**que** le tissu de monofilaments est tissé avec des pores de dimension définie homogènes, dans lequel les dimensions des pores ne divergent pas plus de 10 % les unes des autres.

13. Procédé servant à fabriquer un matériau pour pansement selon l'une quelconque des revendications 1 à 12, où
- un tissu de monofilaments biocompatible avec une surface stable lisse est formé à partir de fils de monofilaments en PET en tant qu'un premier matériau en bande, dans lequel les fils de monofilaments en PET présentent un diamètre de dimensions comprises entre 20 µm et 500 µm,
- un élément de recouvrement est formé en tant que deuxième matériau en bande,
- le premier matériau en bande et le deuxième matériau en bande sont rassemblés, dans lequel le tissu de monofilaments biocompatible est prévu en tant que couche de support avec une face supérieure opposée à la plaie, sur laquelle l'élément de recouvrement est appliqué et est relié de manière solidaire à la couche de support par un soudage par points, et
- le matériau pour pansement est fabriqué sous la forme d'une bande.
